# EUROPÄISCHE PATENTSCHRIFT

(11) **EP 3 580 727 B1**
(45) Veröffentlichungstag und Bekanntmachung des Hinweises auf die Patenterteilung: **24.03.2021**
(21) Anmeldenummer: 18703760.1
(22) Anmeldetag: 05.02.2018
(51) Int. Cl.: G06T 11/00, G06T 19/20

(54) **PROJEKTION VON GEGENSTÄNDEN IN CT-RÖNTGENBILDER**
PROJECTION OF OBJECTS IN CT X-RAY IMAGES
PROJECTION D'OBJETS DANS DES IMAGES PAR RAYONS X TDM

(30) Priorität: 08.02.2017 DE 102017102441
(43) Veröffentlichungstag der Anmeldung: 18.12.2019
(73) Patentinhaber: Smiths Heimann GmbH, 65205 Wiesbaden (DE)
(72) Erfinder: DURZINSKY, Markus, 39104 Magdeburg (DE); MÖRIG, Marc Andreas, 39104 Magdeburg (DE); KÖNIG, Sebastian, 65205 Wiesbaden (DE)
(74) Vertreter: Klunker IP Patentanwälte PartG mbB
(86) Internationale Anmeldenummer: PCT/EP2018/052806
(87) Internationale Veröffentlichungsnummer: WO 2018/146047

(56) Entgegenhaltungen:
- US-A1- 2014 161 333
- NAJLA MEGHERBI ET AL: "Radon transform based automatic metal artefacts generation for 3D threat image projection", VISUAL COMMUNICATIONS AND IMAGE PROCESSING; 20-1-2004 - 20-1-2004; SAN JOSE, Bd. 8901, 16. Oktober 2013 (2013-10-16), Seite 89010B, XP055224962, DOI: 10.1117/12.2028506 ISBN: 978-1-62841-730-2 in der Anmeldung erwähnt

## Beschreibung

Die vorliegende Erfindung betrifft allgemein, beispielsweise für Röntgeninspektionsanlagen an Sicherheitskontrollstellen an Flughäfen erzeugte, Test-Röntgenbilder von Inspektionsobjekten, wobei in ein Test-Röntgenbild ein Gegenstand eingefügt wurde. Im Besonderen betrifft die Erfindung die Erzeugung von Test-Röntgenbildern basierend auf mittels Computertomographie erzeugter dreidimensionaler Röntgenbilder oder daraus abgeleiteter zweidimensionaler Röntgenbilder eines Inspektionsobjekts und hierbei besonders den Aspekt der Erzeugung realistischer und plausibler Metallartefakte, die aufgrund von im Inspektionsobjekt enthaltenen Metallteilen und Metallanteilen des einzufügenden Gegenstands sowie deren gegenseitige Einflüsse zurückzuführen sind.

### Hintergrund der Erfindung

Das Einfügen eines Gefahrengegenstands in ein Röntgenbild ist als Bildprojektion gefährlicher Gegenstände ("Threat Image Projection", TIP) bekannt; wie beispielsweise von V. Cutler et al. beschrieben in "Use of threat image projection (TIP) to enhance security performance", 43. International Carnahan Conference on Security Technology, 5-8 Oktober 2009, S. 46-51, DOI: 10.1109/CCST.2009.5335565. Ein Röntgenbild eines realen Gepäckstücks, in das ein fiktiver Gefahrgegenstand so eingefügt wurde, als ob sich der fiktive Gefahrengegenstand tatsächlich in dem realen Gepäckstück befindet, wird als fiktives Gefahrenbild ("Fictitious Threat Image", FTI) bezeichnet.

DE 10 2014 109 214 A1, US 6 899 540 B1 und US 5 243 693 A zeigen Beispiele für Computersysteme zur Ausbildung und Überprüfung von Bedienpersonen von Röntgeninspektionsanlagen zur Gepäckkontrolle.

Die bekannten TIP-Funktionen betreffen im Wesentlichen 2D-Test-Röntgenbilder, wie sie üblicherweise von Röntgeninspektionsanlagen, die nach dem Line-Scanner-Prinzip arbeiten, bei dem ein Inspektionsobjekt zeilenweise mit Röntgenstrahlen durchstrahlt wird und basierend auf dabei erfassten Intensitätswerten der Röntgenstrahlen nach Durchlaufen des Inspektionsobjekts zugehörige Zeilen eines 2D-Röngenbilds erzeugt werden.

Mittlerweile werden auch Röntgeninspektionsanlagen, die nach dem Prinzip der Computertomographie (CT) arbeiten, bei der zerstörungsfreien Inspektion von Inspektionsobjekten eingesetzt. EP 1 585 995 A1 zeigt z. B. ein entsprechendes Inspektionssystem und Inspektionsverfahren für Gepäck.

CT-Röntgenbilder von Inspektionsobjekten, die Metallteile enthalten, zeigen üblicherweise sogenannte Metallartefakte. Gefahrengegenstände, wie z.B. Waffen oder Sprengvorrichtungen, enthalten üblicherweise Metall, z.B. Metallbestandteile, oder bestehen überwiegend oder ganz aus Metall. Entsprechend muss ein CT-FTI realistische und plausible Metallartefakte aufweisen, die von ggf. bereits im Inspektionsobjekt vorhandenen Metallgegenständen sowie Metall des fiktiven Gefahrengegenstands hervorgerufen würden. Anderenfalls wird das CT-FTI aufgrund unrealistischer oder unplausibler Metallartefakte als FTI erkannt.

Die Bildgebung in der CT basiert im Prinzip auf an einem Detektor erfassten Radondaten, d.h., der erfassten Intensität und der Richtung eines einem Detektor zugeordneten Röntgenstrahls. Jeder Röntgenstrahl wird beim Durchlaufen eines Inspektionsobjekts und der dabei im Strahlengang enthaltenen Materialien abgeschwächt. Bei der computergestützten Bildrekonstruktion kann für jedes Volumenelement (Voxel) eines Inspektionsobjekts als eine Materialeigenschaft der Abschwächungskoeffizient ermittelt werden. Basierend auf diesen für alle Voxel vorliegenden Abschwächungsdaten kann ein entsprechendes 3D-Röntgenbild und/oder können 2D-Schnittbilder errechnet werden.

Metallobjekte weisen einen sehr hohen Abschwächungskoeffizienten auf, sodass es ab einer bestimmten Metalldicke quasi zur Totalabsorption kommen kann, weil das am Detektor erfasste Nutzsignal dann z. B. unter der Rauschschwelle liegt und nicht mehr weiter differenziert werden kann. An scharfen Kanten zwischen einem Metallobjekt und seiner Umgebung kann die Änderung der Abschwächungskoeffizienten besonders hohe Werte annehmen. Werden an derartigen Übergängen Röntgenstrahlen von einem gemeinsamen Detektorelement erfasst, kommt es zu einer Mittelung der erfassten Intensitäten, wobei der resultierende Intensitätswert dem jeweiligen Detektorelement zugewiesen wird. Da der Logarithmus einer linearen Mittelung nicht der Summe der Logarithmen entspricht, ergibt sich ein mittlerer Abschwächungskoeffizient, der nicht zu den mittleren Intensitäten der erfassten Röntgenstrahlen passt. Darauf zurückzuführende Bildartefakte werden als Partialvolumeneffekt bezeichnet. Des Weiteren ist das Spektrum einer Röntgenröhre nicht monochromatisch. Die Strahlenabsorption der vom Röntgenstrahl durchlaufenen Materialien ist Energie abhängig. Röntgenstrahlen mit niedriger Energie werden stärker absorbiert als Röntgenstrahlen mit hoher Energie. Dies führt zu einer Erhöhung der mittleren Energie des Gesamtspektrums. Dies tritt bei allen Materialien auf, aber bei Metallen deutlich stärker. Darauf zurückzuführende Bildartefakte werden als Aufhärtungsartefakte bezeichnet.

Die vorstehend beschriebenen Effekte führen zu Unstimmigkeiten bei den mittels Durchstrahlen des Inspektionsobjekts erfassten Radondaten. Da die bekannte Bild-Rekonstruktion mittels der gefilterten Rückprojektion von idealen Verhältnissen ausgeht, werden die erfassten Radondaten bei der Rekonstruktion über den Bildbereich "verschmiert". Die auf Metall im Inspektionsobjekt zurückzuführenden Effekte wirken sich so auf das gesamte Bild aus. D.h., Metallartefakte in CT-Röntgenbildern werden im Wesentlichen hervorgerufen durch die vorstehend diskutierten Abweichungen der zur Rekonstruktion der Voxel-Daten aus den erfassten Radondaten vom idealen mathematischen Modell. Metallartefakte zeigen sich beispielsweise als dunkle oder weiße Linien, die von einem Metallteil ausgehen und besonders zwischen verschiedenen Metallteilen verlaufen.

Im Bereich der medizinischen Anwendungen der Computertomographie sind Metallartefakte als unerwünschte Bildstörungen bekannt. Beispielsweise schlägt T. Koehler et al. in "A New Method for Metal Artifact Reduction in CT", veröffentlich in "The International Conference in X-ray Computed Tomography", Salt Lake City, Utah, USA, 2011 ein Verfahren zur Entfernung von Metallartefakten aus CT- Röntgenbildern vor.

Es ist bekannt, dass die Intensität und Ausrichtung der auftretenden Metallartefakte abhängen von der Anzahl der Metallteile, der Größe der Metallteile und der Anordnung der Metallteile im Inspektionsobjekt zueinander. Die Projektion eines Gegenstands mit Metallanteilen in ein 3D-CT-Röntgenbild erfordert somit nicht nur das Hinzufügen von Metallartefakten, die der Gefahrengegenstand allein hervorrufen würde, sondern auch solcher Metallartefakte, die aufgrund von Zusammenwirken des Metalls des Gegenstands und des im Gepäckstück bereits befindlichem Metalls auftreten. Gleichfalls sollten Metallartefakte im Gefahrengegenstand selbst sichtbar sein, wenn der Gefahrengegenstand nicht Metall enthält.

Für die Projektion eines Gegenstands in ein CT-Röntgenbild wurde von N. Megherbi et al. ein Verfahren vorgeschlagen, das dies leisten soll. Das bekannte Verfahren ist beschrieben in Najla Megherbi et al. "Fully automatic 3D Threat Image Projection: Application to densely cluttered 3D Computed Tomography baggage images", 2012, 3rd International Conference on Image Processing Theory, Tools and Applications (IPTA), p. 153-159 sowie in N. Megherbi et al., "Radon transform based metal artefacts generation in 3D threat image projection", veröffentlicht in Proc. SPIE Optics and Photonics for Counterterrorism, Crime Fighting and Defence, volume 8901, p. 1-7, SPIE, Oktober 2013, DOI: 10.1117/12.2028506.

Bei dem bekannten Verfahren sollen Metallartefakte, die bei einem realen Gepäckstück durch Metall des Gegenstands sowie ggf. durch dessen Interaktion mit bereits im Gepäckstück vorhandenem Metall im CT-Röntgenbild des Gepäckstücks hervorgerufen würden, im Wesentlichen durch eine Manipulation der Sinogramme des 3D-CT-Röntgenbilds des Gepäckstücks im Radonraum und anschließende Rücktransformation in den Bildraum hinzugefügt werden; die Radontransformierte einer Ebene (Slice) von CT-Daten aus dem Bildraum wird hier - in Anlehnung an den im Englischen verwendeten Begriff - als "Sinogramm" bezeichnet. Der Gegenstand selbst wird in das Gepäckstück im Bildraum eingefügt, indem den Voxel im 3D-CT-Röntgenbild des Gepäckstücks, die dem einzufügenden Gegenstand zugeordnet werden, der jeweilige Abschwächungskoeffizient des zugehörigen Voxel des einzufügenden Gegenstands zugeschrieben werden.

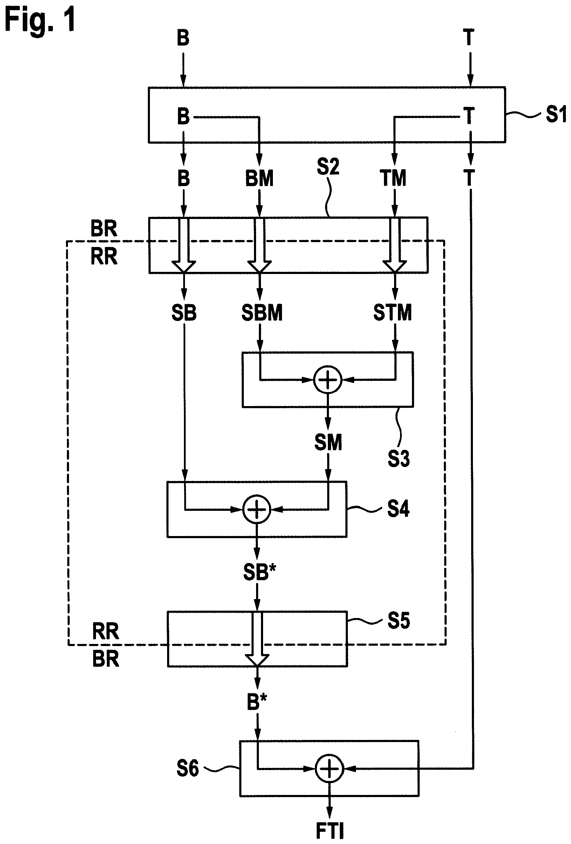

Figur 1 zeigt ein schematisches Flussdiagramm des bekannten Bildverarbeitungsverfahrens von Megherbi et al.

In einem ersten Schritt S1 werden für ein Inspektionsobjekt, z.B. einem Gepäckstück ("bag", B), erste CT-Daten B und zugehörige zweite CT-Daten BM ("bag metall", BM), die freigestellte, im Gepäckstück bereits enthaltene, Metallteile ("metall", M) repräsentieren, erzeugt. Weiter werden dritte CT-Daten TM ("threat metall", TM), die freigestellte Metallteile eines Gefahrengegenstands ("threat", T), z.B. eine Waffe, der in die ersten CT-Daten B eingefügt werden soll, repräsentieren, basierend auf vierten CT-Daten T des Gefahrengegenstands erzeugt. Ergebnis des Schritts S1 sind die ersten CT-Daten B, die zweiten CT-Daten TM, die dritten CT-Daten BM und die vierten CT-Daten T.

In einem zweiten Schritt S2 werden jeweils mittels der bekannten Radontransformation Ebenen (Slices) aus den ersten CT-Daten B, den zweiten CT-Daten BM und den dritten CT-Daten TM aus dem Bildraum BR in den Radonraum RR transformiert. Ergebnis des Schritts S2 sind erste Sinogramm-Daten SB erster Sinogramme für die ersten CT-Daten B, zweite Sinogramm-Daten SBM zweiter Sinogramme für die zweiten CT-Daten BM und dritte Sinogramm-Daten STM dritter Sinogramme für die dritten CT-Daten TM.

Die an sich bekannte "Radontransformation" transformiert Bilder f(x, y) im Bildraum BR (x, y) in den Radonraum RR (α, s). Dabei wird für jeden Winkel α das Linienintegral der Funktion f(x, y) entlang aller Geraden s der x-y-Ebene bestimmt, wobei für jede dieser Geraden s die Radon-Transformierte Rf einer Projektion der Funktion f(x, y) auf eine Senkrechte zur Geraden s entspricht. Die Überlagerung aller Projektionen (Linienintegrale) führt zum Sinogramm, einem 2D-Datensatz. Daraus folgt, der Radonraum RR ist damit zunächst nur zweidimensional. Wird dieses Prinzip auf jede Ebene des 3D-Volumens eines 3D-CT-Röntgenbildes angewendet, werden entsprechend viele Sinogramme erhalten, nämlich eines für jede Ebene. Die Bezeichnung "Radontransformation" wird hier immer als die "Vorwärtstransformation" vom Bildraum BR in den Radonraum RR verstanden. Die Umkehrung der Radontransformation wird "Radonrücktransformation", "inverse Radontransformation" oder auch "gefilterte Rückprojektion" (da die Implementierung im Wesentlichen aus zwei Schritten besteht, nämlich der Filterung und der Transformierung) genannt.

In einem dritten Schritt S3 werden die zweiten Sinogramm-Daten SBM mit entsprechenden dritten Sinogramm-Daten STM zu jeweiligen vierten Sinogramm-Daten SM addiert. Ergebnis des Schritts S3 sind die vierten Sinogramm-Daten SM für vierte Sinogramme, die alle Metallanteile, nämlich die bereits im Gepäckstück enthaltenen Metallanteile sowie die fiktiven Metallanteile des hinzuzufügenden Gefahrengegenstands, repräsentieren.

In einem vierten Schritt S4 werden mittels der vierten Sinogramm-Daten SM in den jeweils zugehörigen ersten Sinogramm-Daten SB die von Metallteilen beeinflussten Stellen identifiziert und - quasi in Umkehrung eines für medizinische CT-Systeme bekannten Verfahrens zur Reduktion von Metallartefakten - manipuliert. Die vierten Sinogramm-Daten SM dienen dabei als Maske, um festzustellen, ob auf einem bestimmten Sinogramm-Strahl in dem Inspektionsobjekt mit dem fiktiv eingefügtem Gefahrengegenstand Metall liegt oder nicht. Ein Sinogramm-Strahl ist dabei die Linie, in welche die Intensitätsinformationen eines bestimmten Punktes in der zugehörigen (Bild-)Ebene (Slice) im Bildraum BR eingehen. Die ersten Sinogramm-Daten SB werden an den so identifizierten Stellen derart modifiziert, dass die Aufhärtung durch Metall simuliert wird. Dazu werden die jeweiligen Werte der als von Metall beeinflusst identifizierten Bildpunkte in den Sinogrammen der ersten Sinogramm-Daten SB erhöht, indem diese näher an einen Maximalwert herangebracht werden. Diese lokalen Modifikationen in den ersten Sinogramm-Daten SB soll bei der Rücktransformation in den Bildraum BR die gewünschten Metallartefakten hervorrufen. D.h., an allen Stellen in den ersten Sinogramm-Daten SB, welche von Metall aus dem Inspektionsobjekt (zweite Sinogramm-Daten SBM) oder Metall aus dem Gefahrenobjekt (dritte Sinogramm-Daten STM) betroffen sind, werden die ersten Sinogramm-Daten SB des Inspektionsobjekts modifiziert. Ergebnis des vierten Schritts S4 sind modifizierte erste Sinogramm-Daten SB*, die für realistische und plausible Metallartefakte jeweils die notwendigen Anomalien enthalten, wie sie in ersten Sinogramm-Daten SB realer erster CT-Daten aufgrund dort tatsächlich enthaltener Metallteile enthalten wären.

Die Erfinder haben es als problematisch erkannt, dass die ersten Sinogramm-Daten SB der ursprünglichen ersten CT-Daten bereits Anomalien aufweisen, welche ggf. bereits in den ersten CT-Daten B enthaltene Metallartefakte repräsentieren. Im bekannten Verfahren werden diese Stellen in den ersten Sinogramm-Daten SB ebenfalls modifiziert, wodurch bereits vorhandene Metallartefakte unrealistisch verstärkt werden.

In einem fünften Schritt S5 werden die modifizierten vierten Sinogramm-Daten SB* mittels der Radonrücktransformation in den Bildraum BR zurücktransformiert. Ergebnis des Schritts S5 sind modifizierte erste CT-Daten B* des Inspektionsobjekts, welches alle benötigten Metattartefakten aufweisen sollten, nämlich Artefakte für bereits im Inspektionsobjekt enthaltene Metallanteile, für Metall des einzufügenden Gefahrengegenstands und die Artefakte zwischen all diesen Metallanteilen.

In einem sechsten Schritt S6 wird in die modifizierten ersten CT-Daten B*, die den Gefahrengegenstand selbst noch nicht enthalten, die vierten CT-Daten T eingefügt. Dazu werden Abschwächungskoeffizienten der Voxel der modifizierten ersten CT-Daten B* durch entsprechende Abschwächungskoeffizienten zugehöriger Voxel der vierten CT-Daten T des einzufügenden Gefahrengegenstands ersetzt. Das Ergebnis des Schritts S6 sind CT-Ergebnisbild-Daten FTI zur Ableitung eines fiktiven Gefahrenbilds.

Beim bekannten Verfahren wird die Bildqualität des Ergebnisbilds wahrnehmbar verändert. Das Röntgenbild wirkt weichgezeichnet und weniger detailreich. Zusätzlich wirken beim bekannten Verfahren die bereits in den ursprünglichen ersten CT-Daten des Gepäckstücks vorhandene Metallartefakte unnatürlich verstärkt. Diese, wenn auch geringfügigen Unterschiede in der Bildqualität im Vergleich zu echten Röntgenbildern fallen, obwohl es sich um vergleichsweise winzige Abweichungen handelt, auf, sodass die Ergebnisbilder in der Praxis nicht zur Überprüfung und Ausbildung von Bedienpersonen einsetzbar sind.

### Offenbarung der Erfindung

Aufgabe der Erfindung ist es, ein Erzeugungsverfahren und eine entsprechende Erzeugungseinrichtung zum Einfügen eines fiktiven Zielgegenstands in Röntgenbilder, die mit einer CT-Röntgeninspektionsanlage erzeugt wurden, vorzuschlagen, wobei die vorstehend im Zusammenhang mit dem bekannten Verfahren diskutierten Nachteile zumindest teilweise vermieden oder wenigstens reduziert sein sollen.

Mit dem Erzeugungsverfahren und der entsprechenden Erzeugungseinrichtung sollen Test-Röntgenbilder von Inspektionsobjekten, in die ein von einer Bedienperson aufzufindender fiktiver Zielgegenstand eingefügt wurde, beispielsweise für Trainings- und/oder Leistungsüberprüfungszwecke, erzeugt werden. Bevorzugt werden die Test-Röntgenbilder basierend auf mittels CT erzeugter CT-Daten als 3D-Bilder oder 2D-Bilder eines Inspektionsobjekts abgeleitet oder erzeugt.

Die erzeugten Test-Röntgenbilder sollten bevorzugt sowohl realistische als auch plausible Metallartefakte aufweisen, die aufgrund von im Inspektionsobjekt enthaltenen Metallteilen und Metallanteilen des einzufügenden fiktiven Zielgegenstands sowie deren gegenseitige Beeinflussungen zurückzuführen sind.

Bereits in einem Röntgenbild enthaltene Metallartefakte sollten bevorzugt nicht unrealistisch verstärkt werden.

Darüber hinaus sollten die Test-Röntgenbilder vom Erzeugungsverfahren möglichst nicht wahrnehmbar in der Bildqualität beeinflusst werden.

Die Aufgabe wird mit den Merkmalen der unabhängigen Ansprüche gelöst. Weitere Ausführungsbeispiele und vorteilhafte Weiterbildungen sind in den sich jeweils anschließenden Unteransprüchen definiert. Merkmale und Details, die in Zusammenhang mit dem erfindungsgemäßen Erzeugungsverfahren beschrieben sind, sind selbstverständlich auch in Zusammenhang mit der erfindungsgemäßen Erzeugungseinrichtung gültig und umgekehrt. Daher wird zur Meidung von Wiederholungen bezüglich der Offenbarung der einzelnen Aspekte wechselseitig Bezug genommen.

Im Hinblick auf das eingangs im Zusammenhang mit der Figur 1 beschriebene bekannte Verfahren sowie dessen Nachteile wird ein erfindungsgemäßes verbessertes Erzeugungsverfahren sowie eine entsprechende Erzeugungseinrichtung zur Erzeugung von Test-Röntgenbildern vorgeschlagen.

Das neue Verfahren vermeidet eine unrealistische Verstärkung von in den ersten CT-Daten B bereits vorhandenen Metallartefakten als auch eine Reduktion der Bildschärfe eines aus den ersten CT-Daten B gewonnenen Röntgenbilds. Der Begriff "Bildschärfe" wird hier als Maß für den lokalen Kontrast verstanden, d.h., wie stark sich die Abschwächungskoeffizienten benachbarter Voxel im Wert unterscheiden können. Wenn CT-Daten mittels der Radontransformation aus dem Bildraum in ein Sinogramm im Radonraum transformiert werden und von dort wieder zurück transformiert werden, führt dies bei einem aus den CT-Daten abgeleiteten Röntgenbild zu einer Verringerung im lokalen Kontrast (Bildschärfe). D.h. ein Ergebnisbild wirkt im Vergleich zum Original weichgezeichnet, weniger detailreich, mithin weniger scharf. Dies wird beispielsweise dadurch verursacht, dass sich ein Materialeigenschaftswert, z. b. ein Abschwächungskoeffizient und/oder ein Materialdichtewert und/oder eine Kernladungszahl, benachbarter Voxel durch die zweimalige Transformation im Wert angenähert haben. Dadurch wird der lokale Kontrast reduziert. Die Reduzierung des lokalen Kontrasts ist als merkliche Reduktion der Bildschärfer wahrnehmbar, das Bild wirkt "verwaschen" oder "weichgezeichnet". Im Vergleich zum bekannten Verfahren, bei dem hinzuzufügende Artefakte in die ersten Sinogramm-Daten SB erster Sinogramme des Inspektionsobjekts addiert werden, werden beim hier vorgeschlagenen neuen Verfahren nur die tatsächlich fehlenden Artefakt-Sinogramm-Daten, welche die hinzuzufügenden (Metall-) Artefakte A repräsentieren, im Radonraum RR berechnet und nach Rücktransformation in den Bildraum BR in die ursprünglichen ersten CT-Daten B eingefügt.

Um dies zu erreichen, wird von den Erfindern vorgeschlagen, neue vierte Sinogramm-Daten SM** (entsprechend vierten Sinogrammen) zu erzeugen, die im Wesentlichen einer logischen Differenzbildung der dritten Sinogramm-Daten STM (entsprechend dritten Sinogrammen) minus der entsprechenden zweiten Sinogramm-Daten SBM (entsprechend zweiten Sinogrammen) entsprechen. Im Ergebnis enthalten die neuen vierten Sinogramm-Daten SM** nur die Punkte, für die ein entsprechender Punkt in einem zugehörigen dritten Sinogramm ungleich Null (STM<>0) und ein entsprechender Punkt im zugehörigen zweiten Sinogramm gleich Null (SBM==0) sind. Mittels der neuen vierten Sinogramm-Daten SM** werden die Punkte in den ersten Sinogramm-Daten SB (entsprechend ersten Sinogrammen) identifiziert, um den Intensitätswert dieser Punkte so zu verändern/manipulieren zu können, dass der Einfluss des in das Inspektionsobjekt fiktiv einzufügenden Metalls in den ersten Sinogramm-Daten SB simuliert wird. So wird im Ergebnis vermieden, dass die bereits in den ersten Sinogramm-Daten SB enthaltenen Artefakt-Informationen, welche die bereits im ersten Röntgenbild B vorhandenen Metallartefakte repräsentieren, im weiteren Verfahren unrealistisch verstärkt werden.

Im neuen Verfahren werden keine modifizierten ersten Sinogramm-Daten SB*, sondern ausschließlich neue Artefakt-Sinogramm-Daten SA erzeugt, die nur die tatsächlich hinzuzufügenden Metallartefakte aufgrund Metalls des Zielgegenstands und des Zusammenwirkens mit ggf. vorhandenen Metall repräsentieren. Dies wird erreicht, indem mit den neuen vierten Sinogramm-Daten SM** quasi als Maske diejenigen Bildpunkte in den entsprechenden ersten Sinogramm-Daten SB identifiziert werden, die zu einem entsprechenden Artefakt-Sinogramm-Daten SA gehören. Alle anderen Bildpunkte in den ersten Sinogramm-Daten SB gehen nicht in die zu berechnenden Artefakt-Sinogramm-Daten SA ein. Die an den so identifizierten Bildpunkten in den ersten Sinogramm-Daten SB vorliegenden Intensitätswerte werden gemäß einer empirisch ermittelten Funktion so manipuliert, dass eine zusätzliche Aufhärtung durch das fiktiv hinzugefügte Metall des Zielgegenstands für durch das Inspektionsobjekt laufende Röntgenstrahlen simuliert wird.

Die so im Radonraum RR berechneten Artefakt-Sinogramm-Daten SA für hinzuzufügende Metall-Artefakte werden in den Bildraum BR transformiert und können dann in die ursprünglichen, d.h. unveränderten, ersten CT-Daten B eingefügt werden. D.h., bei dem neuen Verfahren werden nicht vollständige erstes Sinogramm-Daten SB* mit durch Manipulation im Radonraum hinzugefügte Artefakten zurück in den Bildraum BR transformiert. Die ursprünglichen ersten CT-Daten werden ausschließlich im Bildraum durch Hinzufügen der CT-Artefakt-Daten A und der vierten CT-Daten T des Zielgegenstands bearbeitet. Dadurch bleibt die Bildschärfe der aus den modifizierten ersten CT-Daten B abgeleitete 3D- und/oder 2D-Röntgenbilder gegenüber einem aus den ursprünglichen unveränderten ersten CT-Daten B abgeleitetem Röntgenbild unverändert.

Eine besondere Weitebildung des Verfahren betrifft das Einfügen der vierten CT-Daten T, die den fiktiven Zielgegenstand repräsentieren, sowie der CT-Artefakt-Daten A, welche die hinzuzufügenden Metallartefakte repräsentieren, in die ersten CT-Daten B des Inspektionsobjekts.

Bevorzugt werden die vierten CT-Daten T eines Zielgegenstands vorab erzeugt. Dazu kann jeweils ein Zielgegenstand mittels eines Stützmaterials geringer Dichte, beispielsweise Styropor, in Position gehalten und dessen vierte CT-Daten T mittels einer CT-Röntgeninspektionsanlage erfasst werden. Das Stützmaterial kann sich dazu unter dem Zielgegenstand befinden oder diesen homogen vollständig einschließen. Die so erhaltenen vierten CT-Daten T der Zielgegenstände können in einer Datenbank zur späteren Verwendung vorgehalten werden.

In der Weiterbildung werden die vierten CT-Daten T des einzufügenden Zielgegenstands und/oder die dazu berechneten realistischen und plausiblen CT-Artefakt-Daten A direkt ohne ein aufwändiges Freistellen des Zielgegenstands verwendet und in die ersten CT-Daten B des Inspektionsobjekts eingefügt. Dabei werden nicht lediglich Materialeigenschaftswerte eines Voxel in den ersten CT-Daten B durch entsprechende Materialeigenschaftswerte in den vierten CT-Daten T des Zielgegenstands oder in den hinzuzufügende CT-Artefakt-Daten A ersetzt, sondern in Abhängigkeit vom jeweiligen Materialeigenschaftswerts eines einzufügenden Voxel in einem Übergangsbereich eine "Verblendung" oder "gewichtete Kombination" der beiden Materialeigenschaftswerte durchgeführt. Beispielsweise können der eine Wert mit 20% und der andere Wert mit 80% in den neuen Wert eingehen; selbstverständlich sind auch andere Gewichtungen möglich.

Beispielsweise wird in einer Ausführung mittels eines geeigneten vorbestimmten Schwellwerts in den vierten CT-Daten T und/oder in den CT-Artefakt-Daten A der hinzuzufügenden Metallartefakte zwischen ersten Voxel, deren Materialeigenschaftswert unter dem vorbestimmten Schwellwert liegen, zweiten Voxel, deren Materialeigenschaftswert in einem vorbestimmten Bereich über dem vorbestimmten Schwellwert liegt, und dritten Voxel, deren Materialeigenschaftswert über diesem vorbestimmten Bereich liegt, unterscheiden. Die ersten Voxel werden als nicht zum Zielgegenstand oder nicht als Artefakte, beispielsweise als Hintergrund interpretiert. Das ist z.B. bei den vierten CT-Daten T das Stützmaterial. D.h., erste Voxel werden ignoriert. Zweite Voxel mit einem Materialeigenschaftswert aus dem vorbestimmten Bereich werden beim Einfügen in die ersten CT-Daten B jeweils mit dem Materialeigenschaftswert des dort entsprechenden Voxel "verblendet" (gewichtet kombiniert). Bei dritten Voxel werden beim Einfügen in die CT-Daten B des Inspektionsobjekts der Materialeigenschaftswert des örtlich entsprechenden Voxel durch den Materialeigenschaftswert des Voxel des dritten Voxel ersetzt.

Die vorstehend beschriebene Klassifizierung ermöglicht auch eine für das oben geschilderte Verfahren zur Erzeugung der Artefakte geeignete Freistellung der Metallteile im Inspektionsobjekt oder des Metalls eines einzufügenden Zielgegenstands. D.h., das neue Verfahren kann automatisch Metallanteile des Zielgegenstands und Metallanteilen des Inspektionsobjekts oder hinzuzufügenden Artefakte mittels der vorbestimmten Schwellwerte erkennen und von nichtmetallischen Bestandteilen unterscheiden.

Ein erster Aspekt der Erfindung betrifft ein Erzeugungsverfahren für Test-Röntgenbitder. Das Verfahren weist im Prinzip auf:
Bereitstellen erster Computertomografie-, CT-, Daten B, die ein Inspektionsobjekt repräsentieren, zweiter CT-Daten BM, die Inspektionsobjekt enthaltene Metallanteile repräsentieren, dritter CT-Daten TM, die Metallanteile eines Zielgegenstands repräsentieren, der in die ersten CT-Daten B eingefügt werden soll;
Transformieren der ersten, zweiten und dritten CT-Daten B, BM, TM aus dem Bildraum in entsprechende erste Sinogramm-Daten SB, zweite Sinogramm-Daten SBM und dritte Sinogramm-Daten TM im Radonraum;
Berechnen von Artefakt-Sinogramm-Daten SA im Radonraum, die Metall-Artefakte repräsentieren, die den ersten CT-Daten B aufgrund des einzufügenden Zielgegenstands hinzuzufügen sind;
Rücktransformieren der Artefakt-Sinogramm-Daten SA aus dem Radonraum in den Bildraum in CT-Artefakt-Daten A, welche die einzufügenden Artefakte repräsentieren; und
Einfügen der CT-Artefakt-Daten A in die ersten CT-Daten B.

Die Inspektionsobjekte und Zielobjekte werden bevorzugt mit einer CT-Röntgeninspektionsanlage gescannt.

Bevorzugt weist das Erzeugungsverfahren weiter auf: Einfügen vierter CT-Daten T, die den Zielgegenstand repräsentieren, in die ersten CT-Daten B, um CT-Testbild-Daten FTI zu erhalten.

Bevorzugt weist das Erzeugungsverfahren weiter auf: Ableiten von CT-Röntgenbildern und/oder 2D-Röntgenbildern des Inspektionsobjekts aus den CT-Testbild-Daten FTI. Die 3D-CT-Röntgenbildern und/oder 2D-Röntgenbildern des Inspektionsobjekts können als Test-Röntgenbilder beispielsweise zur Ausbildung und/oder Überprüfung von Bedienpersonen einer Röntgeninspektionsanlage verwendet werden.

Es sei angemerkt, dass das Erzeugungsverfahren grundsätzlich zur Erzeugung eines bestimmten 2D-Röntgenbilds mit eingefügtem fiktivem Zielgegenstand ausgeführt werden kann. Dazu ist reicht es im Prinzip aus, das Verfahren nur für die betroffene Bildebene, d.h. ein bestimmtes Slice in den ersten CT-Daten B und entsprechend in den zweiten, dritten und vierten CT-Daten BM, TM, und T durchzuführen.

Ein dreidimensionales, 3D-, Computertomografie-, CT-, Röntgenbild, aber auch ein zweidimensionales, 2D-, Röntgenbild sind eine mögliche Darstellung zugehöriger CT-Daten.

CT-Daten können mittels CT für ein gescanntes Objekt, z.B. das Inspektionsobjekt und das einzufügende Zielobjekt, erhalten werden. Beim CT-Scanvorgang werden zunächst Radondaten des Objekts erfasst. Die Radondaten (oder Sinogramm-Daten) können mittels der gefilterten Rückprojektion in CT-Daten des Objekts transformiert werden. D.h., CT-Daten sind im Wesentlichen ein 3D-Datensatz, in dem einzelnen Volumenelementen (Voxel) des Objekts jeweils eine Materialeigenschaft des Materials an der Stelle des Voxel zugeordnet ist.

Sinogramme im Radonraum sind im Wesentlichen eine mögliche Darstellung der Radondaten, welche die beim Scannen eines Objekts mittels CT erfassten Intensitätsinformationenenthalten. Im Prinzip wird bei der CT ein Objekt in einer Ebene (Slice) mit Röntgenstrahlen einer punktförmigen Röntgenquelle aus mehreren Projektionsrichtungen durchstrahlt. Dabei wird für jede Projektionsrichtung in dieser Ebene auf einem gegenüberliegenden Detektor hinter dem Objekt ein zugehöriges Intensitätsprofil, das einer perspektivischen Projektion des Objekts entspricht, erhalten. Dreht man die Röntgenquelle und den Detektor um das Objekt, erhält man entsprechend für die vielen Projektionsrichtungen das jeweils zugehörige Intensitätsprofil. Werden die so erfassten Intensitätsinformationen über der Projektionsrichtung und der Detektorbreite als rechtwinkliges Koordinatensystem aufgetragen erhält man ein Sinogramm als eine mögliche grafische Darstellung der erfassten Radondaten (Sinogramm-Daten).

Für jeden Bildpunkt (p) eines zugehörigen Artefakt-Sinogramms SA kann ein modifizierter Intensitätswert entsprechend der folgenden Vorschrift berechnet werden:

| | | |
|---|---|---|
| (i) | SA(*p*) = (*q µ* - *q* SB(*p*)), | wenn STM(*p*) ≠ 0 und SBM(p) = 0, und |
| (ii) | SA(*p*) = 0, | anderenfalls, |

wobei *q* ein vorbestimmter Wert zwischen 0 und 1, und *µ* der Maximalwert in den zugehörigen zweiten Sinogramm-Daten ist. Der Faktor *q* kann ein Wert zwischen 0,02 und 0.06 sein. Bevorzugt ist der Faktor *q* ein Wert zwischen 0,03 und 0,04. Besonders bevorzugt beträgt der Faktor *q* gleich 0,04.

Das Einfügen von CT-Artefakt-Daten A und/oder vierten CT-Daten T in die ersten CT-Daten B kann aufweisen: Verblenden oder gewichtetes Kombinieren eines Materialeigenschaftswerts, der einem bestimmten Voxel in den ersten CT-Daten B zugeordnet ist, in Abhängigkeit von einem jeweiligen Materialeigenschaftswert, der einem entsprechenden Voxel der einzufügenden CT-Daten A, T zugeordnet ist, wobei dem entsprechenden Voxel und dem bestimmten Voxel sich entsprechende Raumkoordinaten zugeordnet sind.

Bevorzugt weist das Einfügen von CT-Daten A, T in die ersten CT-Daten B auf:
Klassifizieren mittels eines vorbestimmten Schwellwerts in den einzufügenden CT-Daten (T, A) von ersten Voxeln, deren Materialeigenschaftswert unter dem vorbestimmten Schwellwert liegt, von zweiten Voxeln, deren Materialeigenschaftswert in einem vorbestimmten Bereich über dem vorbestimmten Schwellwert liegt, und von dritten Voxeln, deren Materialeigenschaftswert über diesem vorbestimmten Bereich liegt;
Ignorieren der CT-Daten der ersten Voxel;
Verblenden des Materialeigenschaftswerts, der einem bestimmten Voxel der ersten CT-Daten zugeordnet ist, mit dem Materialeigenschaftswert eines entsprechenden zweiten Voxel der einzufügenden CT-Daten; und
Ersetzen des Materialeigenschaftswerts, der einem bestimmten Voxel der ersten CT-Daten zugeordnet ist, durch einen Materialeigenschaftswert der einem entsprechenden Voxel der einzufügenden CT-Daten zugeordnet ist,
wobei im Schritt des Verblendens und im Schritt des Ersetzens dem entsprechenden Voxel und dem bestimmten Voxel sich entsprechende Raumkoordinaten zugeordnet sind.

Bevorzugt weisen die CT-Daten B auf: einen Wert für eine Materialeigenschaft; und Koordinaten zur Bestimmung (des Orts) eines Volumenelements (Voxel), dem der wenigstens eine Wert für eine Materialeigenschaft zugeordnet ist. Der dem jeweiligen Voxel zugeordnete Materialeigenschaftswert ist bevorzugt wenigstens eines von: einem Absorptionskoeffizient, einem Materialdichtewert und einer Kernladungszahl. Bevorzugt sind die Koordinaten zur Bestimmung eines Voxel dreidimensionale, 3D-, Koordinaten, die den Ort des Voxel im Raum identifizieren.

Ein zweiter Aspekt der Erfindung betrifft ein Computerprogrammprodukt mit einem Computerprogramm, das Softwaremittel zur Durchführung eines Verfahrens gemäß dem ersten Aspekt der Erfindung aufweist, wenn das Computerprogramm in einem Automatisierungssystem, beispielsweise ein Computersystem, ausgeführt wird.

Ein dritter Aspekt der Erfindung betrifft eine Erzeugungseinrichtung zur Erzeugung von Test-Röntgenbildern, wobei die Erzeugungseinrichtung aufweist: eine Inspektionsobjektdatenbank zur Speicherung erzeugter erster CT-Daten B von Inspektionsobjekten; eine Zielgegenstanddatenbank mit vierten CT-Daten T und ggf. dritten CT-Daten TM von Zielgegenständen; und eine Bildprojektionseinheit zur Projektion eines Zielgegenstands aus der Zielobjektdatenbank in ein Röntgenbild aus der Inspektionsobjektdatenbank zur Erzeugung eines Test-Röntgenbilds, wobei die Bildprojektionseinheit zur Durchführung eines Verfahrens gemäß dem ersten Aspekt der Erfindung eingerichtet ist. Die Erzeugungseinrichtung weist bevorzugt zur Durchführung des Verfahrens gemäß dem ersten Aspekt ein Automatisierungssystem auf, beispielsweise ein Computersystem.

Die Erzeugungseinrichtung kann weiter eine Aktualisierungseinheit für die Inspektionsobjektdatenbank aufweist. Die Aktualisierungseinheit kann eingerichtet sein, die Inspektionsobjektdatenbank zu aktualisieren, indem erste CT-Daten B, die älter als eine vorbestimmte Zeitdauer sind und/oder für eine bestimmte Anzahl, z. B. einmal, zur Erzeugung von CT-Testbild-Daten FTI verwendet wurde, aus der Inspektionsobjektdatenbank gelöscht oder für eine weitere Verwendung gesperrt werden. Die Bildprojektionseinheit kann weiter eingerichtet sein, Test-Röntgenbilder in einer Test-Röntgenbilderdatenbank zu speichern. Die Aktualisierungseinheit und/oder die Bildprojektionseinheit kann/können eigerichtet sein, CT-Testbild-Daten FTI in der Datenbank zu aktualisieren, indem CT-Testbild-Daten FIT, die älter als eine zweite Zeitdauer sind und/oder für eine bestimmte Anzahl, z. B. einmal, zur Ausbildung oder Überprüfung einer Bedienperson verwendet wurden, gelöscht oder für eine weitere Verwendung gesperrt werden.

Die Erzeugungseinrichtung kann Teil einer Röntgeninspektionsanlage sein oder mit dieser kommunikativ und/oder operativ verbunden sein, um eine "live"-Überprüfung und/oder Ausbildung von Bedienpersonen zu ermöglichen. Bevorzugt werden daher die CT-Testbild-Daten von Inspektionsobjekten mit einer Röntgeninspektionsanlage am jeweiligen Kontrollort erzeugt.

Ein vierter Aspekt der Erfindung betrifft eine zentrale Steuereinheit für wenigstens eine Röntgeninspektionsanlage zur zerstörungsfreien Inspektion eines Inspektionsobjekts, insbesondere eines Gepäckstücks oder sonstigen Versandstücks, mit einer Erzeugungseinrichtung gemäß dem dritten Aspekt, wobei die Steuereinheit bevorzugt über ein Kommunikationsnetzwerk mit der wenigstens einen Röntgeninspektionsanlage verbunden ist.

Die zentrale Steuereinheit weist bevorzugt wenigstens eine Anzeigeeinheit zur Anzeige von Röntgenbildern eines aktuellen Inspektionsobjekts zur Sichtkontrolle durch eine Bedienperson oder zur Anzeige eines Test-Röntgenbitds auf. Bevorzugt ist die zentralen Steuereinheit zu der wenigstens einen Röntgeninspektionsanlage so angeordnet, dass für eine Bedienperson ein aktuell mit der wenigstens einen Röntgeninspektionsanlage zu kontrollierendes Inspektionsobjekt nicht sichtbar ist.

Ein fünfter Aspekt der Erfindung betrifft eine Röntgeninspektionsanlage zur zerstörungsfreien Inspektion eines Inspektionsobjekts, insbesondere eines Gepäckstücks oder sonstigen Versandstücks, die eine Zentrale Steuereinheit gemäß dem vierten Aspekt aufweist oder damit operativ und kommunikativ verbunden ist.

Anhand von Röntgenbildern, die mit einer Röntgeninspektionsanlage erzeugt wurden, kann eine Bedienperson in einem Inspektionsobjekt Zielgegenstände auffinden. Die Röntgeninspektionsanlage kann beispielsweise eine CT-Röntgeninspektionsanlage sein, wie sie an Sicherheitsüberprüfungsstellen, z. B. an Zugängen zu Sicherheitsbereichen oder im Gepäckumschlagsystem, beispielsweise an Flughäfen, eingesetzt werden, um Gepäckstücke und/oder Frachtgüter, die an Bord eines Flugzeugs verbracht werden sollen, manuell oder automatisch zerstörungsfrei zu inspizieren. Röntgeninspektionsanlagen können auch an anderen Kontrollstellen z.B. an Zugängen zu sicherheitsrelevanten Bereichen oder Gebäuden, an Grenzkontrollstellen etc. zur Inspektion von Gegenständen, wie von Personen mitgeführtes Handgepäck oder Poststücke wie Briefe, Päckchen und Pakete, verwendet werden.

Ziel der Inspektionen ist die Entdeckung bestimmter Zielgegenstände. Zielgegenstände können Gegenstände oder Substanzen mit Gefährdungspotential, wie Waffen, Sprengstoffe, Chemikalien etc., d. h. gefährliche Gegenstände sein. Zielgegenstände können auch Gegenstände oder Substanzen sein, von denen zunächst keine unmittelbare Bedrohung ausgeht. Zielgegenstände können z. B. Datenträger, wie z. B. DVDs oder CD-ROMs, Schmuggelgüter, Geld, Drogen etc. sein. Zielgegenstände können auch grundsätzlich ungefährliche aber aus bestimmten Gründen als gefährlich eingestufte Gegenstände sein, z.B. Li-lonen-Batterien. Zielgegenstände können mit einem Einführverbot belegte Lebensmitteln sein. Grundsätzlich können beliebige Gegenstände, z. B. zum Training von Bedienpersonen, als Zielgegenstand definiert werden. Unter einem Zielgegenstand wir damit grundsätzlich ein Gegenstand oder eine Substanz verstanden, die eine Bedienperson in Röntgenbildern eines Inspektionsobjekts erkennen soll. Für diese Zwecke können entsprechende CT-Bilddaten von Zielgegenständen erzeugt und in einer Zielgegenstanddatenbank vorgehalten werden.

Mit dem hier vorgeschlagenen Verfahren können realistische CT-Testbild-Daten FTI erzeugt werden und im laufenden Betrieb zur Überprüfung von Bedienpersonen von Röntgeninspektionsanlagen oder zu deren Ausbildung verwendet werden. Aufgrund der beschriebenen neuartigen Erzeugung der CT-Testbild-Daten FTI enthalten diese sowohl realistische als auch plausible Metallartefakte, wie sie reale CT-Röntgenbilder eines Inspektionsobjekts, das den Zielgegenstand tatsächlich enthält, zeigen würden. Außerdem weisen die mit dem vorgeschlagenen Verfahren erzeugten CT-FTIs keine Veränderungen der Bildschärfe auf, sind somit nicht aus diesem Grund als FTIs erkennbar.

Ein sechster Aspekt der Erfindung betrifft ein Gepäck- oder Versandstückkontrollsystem mit wenigstens einer Röntgeninspektionsanlage und einer zentralen Steuereinheit gemäß dem vierten Aspekt.

### Kurze Beschreibung der Figuren

Weitere Vorteile, Merkmale und Einzelheiten der Erfindung ergeben sich aus der nachfolgenden Beschreibung, in der unter Bezugnahme auf die Zeichnungen Ausführungsbeispiele im Einzelnen beschrieben sind. Dabei können die in den Patentansprüchen und/oder in der Beschreibung erwähnten Merkmale jeweils einzeln für sich oder in beliebiger Kombination wesentlich sein. Ebenso können die vorstehend genannten und die hier weiter ausgeführten Merkmale je für sich oder zu mehreren in beliebigen Kombinationen Verwendung finden. Funktionsähnliche oder identische Bauteile oder Komponenten sind teilweise mit gleichen Bezugszeichen versehen. Die in der Beschreibung der Ausführungsbeispiele verwendeten Begriffe "links", "rechts", "oben" und "unten" beziehen sich auf die Zeichnungen in einer Ausrichtung mit normal lesbarer Figurenbezeichnung bzw. normal lesbaren Bezugszeichen. Die gezeigten und beschriebenen Ausführungsbeispiele sind nicht als abschließend zu verstehen, sondern haben beispielhaften Charakter zur Erläuterung. Die detaillierte Beschreibung dient der Information des Fachmanns, daher werden bei der Beschreibung bekannte Schaltungen, Strukturen und Verfahren nicht im Detail gezeigt oder erläutert.
- Figur 1: zeigt ein schematisches Flussdiagram eines bekannten Verfahrens zum Einfügen fiktiver Gefahrengegenstände in CT-Röntgenbilder.
- Figur 2: zeigt ein schematisches Flussdiagram eines Ausführungsbeispiels für ein erfindungsgemäßes Verfahren zum Einfügen fiktiver Zielgegenstände in CT-Röntgenbilder.
- Figur 3: zeigt ein automatisches Gepäck- oder Versandstückkontrollsystem in schematischer Darstellung als Blockdiagramm.
- Figur 4: zeigt ein Ausführungsbeispiel einer Erzeugungseinrichtung für 3D-CT-Test-Röntgenbilder.
- Figur 5A: zeigt ein original 3D-CT-Röntgenbild eines Gepäckstücks als Beispiel für ein Inspektionsobjekt mit einigen Metallartefakten, die von Metallteilen in dem Gepäckstück verursacht sind.
- Figur 5B: zeigt das 3D-CT-Röntgenbild der Figur 5a, in das ein Gefahrengestand als Zielgegenstand und die erfindungsgemäß erzeugten Metallartefakte eingefügt sind.

### Bevorzugte Ausführungsbeispiele

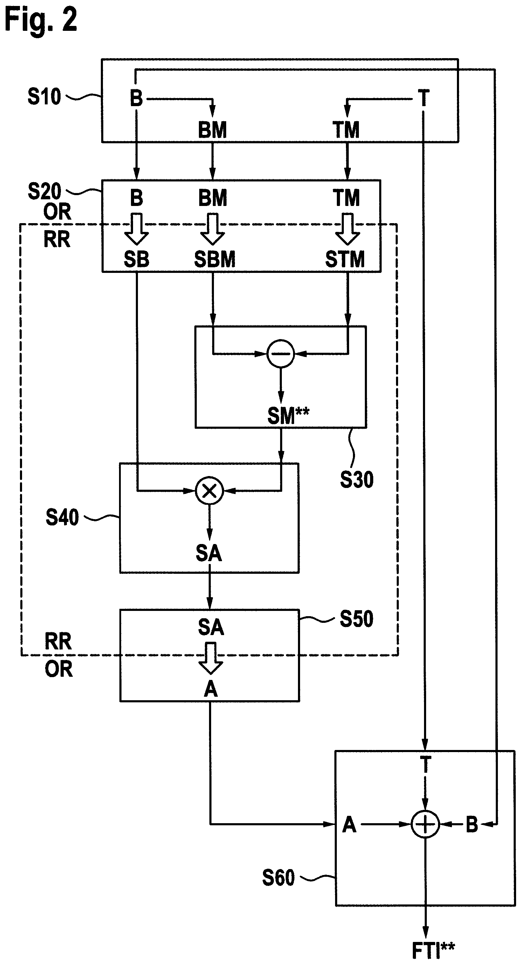

Figur 2 zeigt ein schematisches Flussdiagram eines Ausführungsbeispiels für das hier vorgeschlagene neue Verfahren zum Einfügen fiktiver Zielgegenstände in CT-Röntgenbilder.

In einem ersten Schritt S10 werden erzeugt ein erstes CT-Röntgenbild eines Inspektionsobjekts, d.h. erste CT-Daten B des Inspektionsobjekts, beispielsweise eines Gepäckstücks. Aus den CT-Daten B des Inspektionsobjekts werden ein zugehöriges zweites CT-Röntgenbild erzeugt, d.h. zweite CT-Daten BM, mit freigestellten Metallanteilen, die ggf. im Gepäckstück bereits enthalten sind. Weiter werden ein drittes CT-Röntgenbild erzeugt, d.h. dritte CT-Daten TM, mit freigestellten Metallanteilen eines Zielgegenstands, der in das erste CT-Röntgenbild eingefügt werden soll. Das dritte CT-Röntgenbild, d.h. die dritten CT-Daten TM, werden basierend auf einem vierten CT-Röntgenbild, d.h. vierten CT-Daten T dieses Zielgegenstands erzeugt.

Ergebnis des ersten Schritts S10 sind somit die ersten CT-Daten B, die zweiten CT-Daten BM, die dritten CT-Daten TM und die vierten CT-Daten T.

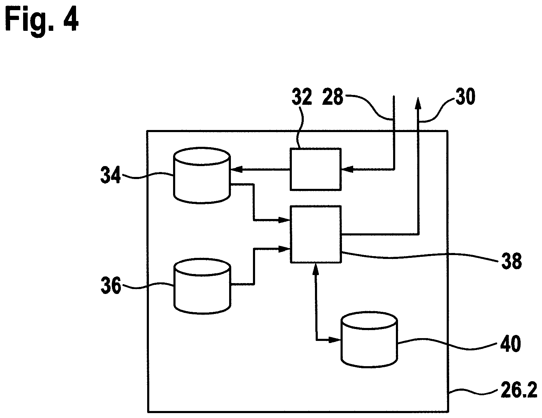

Die vierten und/oder dritten CT-Daten TM, T von Zielgegenständen können bereits vorab erzeugt worden sein und zur weiteren Verwendung in einer Zielgegenstandsdatenbank (vgl. z.B. Bezugszeichen 34 in Figur 4) vorgehalten werden.

In einem zweiten Schritt S20 werden jeweils durch Anwendung der bekannten Radontransformation die ersten CT-Daten B, die zweiten CT-Daten BM und die dritten CT-Daten TM aus dem Bildraum BR in den Radonraum RR transformiert. Dadurch werden entsprechende erste Sinogramm-Daten SB für die ersten CT-Daten B, zweite Sinogramm-Daten SBM für die zweiten CT-Daten BM und dritte Sinogramm-Daten STM für die dritten CT-Daten TM erhalten.

In einem dritten Schritt S30 werden basierend auf zweiten Sinogramm-Daten SBM und jeweils entsprechenden dritten Sinogramm-Daten STM jeweilige neue vierte Sinogramm-Daten SM** erzeugt. Die neuen vierten Sinogramme entsprechen im Wesentlichen einer Kombination im Sinne einer logischen Differenz, um exklusiv Punkte der dritten Sinogramm-Daten STM zu ermitteln. Die neuen vierten Sinogramme enthalten nur die Bildpunkte, für die (ausschließlich) der entsprechende Bildpunkt im zugehörigen dritten Sinogramm ungleich Null ist, d.h. STM<>0 und gleichzeitig der entsprechende Bildpunkt im zugehörigen zweiten Sinogramm gleich Null ist, d.h. SBM==0. Dadurch wird vermieden, dass bereits in den ersten Sinogrammen enthaltene Artefakt-Informationen (=SBM), welche die bereits im ersten 3D-CT-Röntgenbild vorhandenen Metallartefakte repräsentieren, im weiteren Verfahren verstärkt werden. Das Ergebnis des dritten Schritts S30 des neuen Verfahrens sind neue vierte Sinogramm-Daten SM**, die ausschließlich Metallteile des hinzuzufügenden Gefahrengegenstands repräsentieren.

In einem vierten Schritt S40 werden mittels der neuen vierten Sinogramm-Daten SM** die entsprechenden Bildpunkte eines jeweils zugehörigen ersten Sinogramms SB als die von den hinzuzufügenden Metallteilen beeinflussten Stellen identifiziert. An diesen identifizierten Stellen wird die dort jeweils vorliegende Intensitätsinformation manipuliert, um die neuen Artefakt-Sinogramme zu erzeugen. Damit das neue Verfahren keine modifizierten ersten Sinogramme, sondern nur die neuen Artefakt-Sinogramm-Daten SA erzeugt, die nur die tatsächlich hinzuzufügenden Metallartefakte A aufgrund Metalls des Zielgegenstands und der Interaktion mit ggf. vorhandenen Metall repräsentieren, gehen andere Intensitätsinformationen der jeweiligen ersten Sinogramm-Daten SB nicht in das zugehörige neue Artefakt-Sinogramm ein. Dies wird erreicht, indem mit den neuen vierten Sinogramm-Daten SM** als Maske diejenigen Punkte in den ersten Sinogrammen identifiziert werden, die in das neue Artefakt-Sinogramm eingehen, während alle andere Stellen des ersten Sinogramms nicht in das Artefakt-Sinogramm eingehen. Für alle identifizierten Stellen in den ersten Sinogramm-Daten SB wird eine Aufhärtung durch Metall der in den ersten Sinogrammen an diesen Stellen vorhandenen Intensitäten für die Röntgenstrahlen durch das Inspektionsobjekt berechnet.

Die Schritte S30 und S40 werden bevorzugt zusammen ausgeführt. Beispielsweise kann zur Erzeugung eines Artefakt-Sinogramms SA für jeden Bildpunkt *p* ein Intensitätswert SA(p) entsprechend der folgenden erfindungsgemäßen Vorschrift berechnet werden:

| | | |
|---|---|---|
| (i) | *SA*(*p*) = (*q µ* - *q* SB(*p*)), | wenn STM(*p*) ≠ 0 und SBM(p) = 0, und |
| (ii) | SA(*p*) = 0, | anderenfalls, |

wobei *q* ein vorbestimmter Wert zwischen 0 und 1 und *µ* = max(SBM) sind. Die Vorschrift (i) identifiziert und modifiziert solche Bildpunkte des jeweiligen ersten Sinogramms, die mit Metallteilen assoziiert werden können. Die Vorschrift (ii) stellt sicher, dass keine Bildpunkte aus den ersten Sinogrammen, die nicht mit dem fiktiv hinzuzufügenden Metall assoziiert werden können, in das neue Artefakt-Sinogramm eingehen. Der Wert *q* kann ein Werte größer gleich 0,02 und kleiner gleich 0,06 sein. Der Wert *q* ist bevorzugt ein Werte größer gleich 0,02 und kleiner gleich 0,06 In der hier besonders bevorzugten Ausführung ist der Wert *q* gleich 0,04.

In vorteilhafter Weise vermeidet das erfindungsgemäße Verfahren eine Beeinflussung der bereits in dem ersten 3D-CT-Röntgenbild B enthaltenen Metallartefakte, die von den darin bereits enthaltenden Metallteilen verursacht sind. Dazu werden in einem zweiten Modifikationsschritt nur die zusätzlich auf das Metall im Zielgegenstand und der Interaktionen dieses Metalls mit den bereits vorhandenen Metallteilen zurückzuführenden Metallartefakte vom erfindungsgemäßen Verfahren erzeugt.

Ergebnis des vierten Schritts S40 sind damit neuartige Artefakt-Sinogramm-Daten SA, die jeweils die notwendigen Anomalien für die hinzuzufügenden Metallartefakte enthalten, die auch in ersten Sinogramm-Daten SB realer CT-Daten aufgrund tatsächlich enthaltener Metallteile enthalten sind.

In einem fünften Schritt S50 werden die im Radonraum RR berechneten Artefakt-Sinogramm-Daten SA für hinzuzufügende Artefakte in den Bildraum BR rücktransformiert, sodass im Bildraum BR CT-Daten A für die hinzuzufügenden Artefakten erhalten werden.

In einem sechsten Schritt S60 werden die CT-Daten A mit den hinzuzufügenden Artefakten und die vierten CT-Daten T für den hinzuzufügenden Zielgegenstand in die ursprünglichen ersten CT-Daten B eingefügt. Das Ergebnis des sechsten Schritts S60 sind CT-Testbild-Daten FTI für das Inspektionsobjekt mit eingefügtem fiktivem Zielgegenstand. Aus den CT-Testbild-Daten FTI können entsprechende 3D-CT-Röntgenbilder oder entsprechende 2D-Röntgenbilder des Inspektionsobjekts zur weiteren Verwendung abgeleitet werden. Die so erhaltenen 3D-CT-Röntgenbilder oder 2D-Röntgenbilder sind gegenüber solchen, die mit dem bekannten Verfahren erhalten wurden, deutlich betreffend der Plausibilität und realistischen Erscheinung der Metallartefakte sowie der Bildschärfe verbessert.

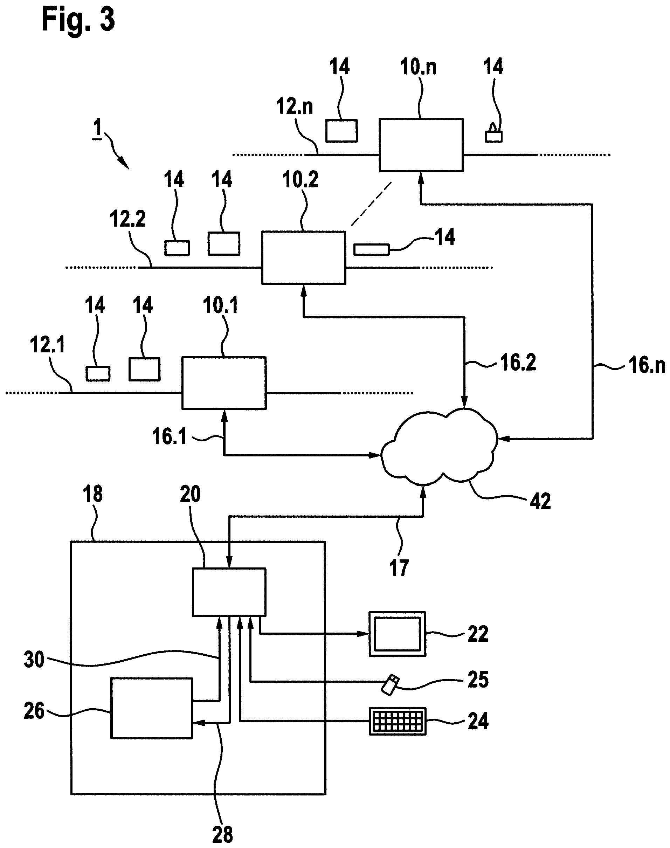

Figur 3 zeigt ein automatisches Gepäck- oder Versandstückkontrollsystem 1 (kurz: Kontrollsystem 1), wie es beispielsweise auf Flughäfen für Level-2 Gepäck- oder Versandstücke eingesetzt wird. Dort werden beispielweise Gepäck- oder Versandstücke als Inspektionsobjekte inspiziert, die von Dritten oder Passagieren zur Verladung in den Laderaum eines Flugzeugs vorgesehen sind. Es sei angemerkt, dass die hier im Zusammenhang mit der Bildprojektion gefährlicher Gegenstände (TIP) diskutierten Maßnahmen grundsätzlich in Verbindung mit allen Ausführungen von Röntgeninspektionsanlagen eingesetzt werden können.

Das Kontrollsystem 1 enthält eine Anzahl n von an sich bekannte CT-Röntgeninspektionsanlagen 10.1, 10.2 und 10.n, die jeweils schematisch mit einem Rechteck angedeutet sind. Auf Transportbändern 12.1, 12.2 und 12.n, die der jeweiligen CT-Röntgeninspektionsanlage 10.1, 10.2 und 10.n zugeordnet sind, werden Gepäck- oder Versandstücke als Inspektionsobjekte 14 zur automatischen Inspektion jeweils durch einer der CT-Röntgeninspektionsanlagen transportiert. Die CT-Röntgeninspektionsanlagen 10.1, 10.2, 10.n arbeiten nach den bekannten CT-Prinzipien und müssen hier nicht näher erläutert werden.

Alle CT-Röntgeninspektionsanlagen 10.1, 10.2, 10.n des Kontrollsystems 1 sind über jeweilige Datenverbindungen 16.1, 16.2 und 16.n über ein Datennetzwerk 42 mit wenigstens einer räumlich von den einzelnen Röntgeninspektionsanlagen 10.1, 10.2 und 10.n abgesetzten zentralen Steuereinheit 18 vernetzt. Die zentralen Steuereinheit 18 selbst ist über eine entsprechende Datenverbindung 17 mit dem Datennetzwerk 42 verbunden.

Die zentrale Steuereinheit 18 ist der Arbeitsplatz einer Bedienperson. Zur Sichtprüfung von Röntgenbildern eines Inspektionsobjekts ist eine Anzeigeeinheit 22 vorgesehen. Auf der Anzeigeeinheit 22 werden beispielsweise, wenn eine der Röntgeninspektionsanlagen 10.1, 10.2, 10.n nicht automatisch über die Unbedenklichkeit eines Inspektionsobjekts 14 entscheiden kann, der Bedienperson das Röntgenbild oder die Röntgenbilder des betreffenden Inspektionsobjekts angezeigt. Das heißt, wenn die in den Röntgeninspektionsanlagen 10.1, 10.2, 10.n implementierten Inspektionsroutinen anhand eines oder mehrerer Röntgenbilder eines aktuellen Inspektionsobjekts 14 nicht mit der notwendigen Sicherheit feststellen können, dass sich kein Zielgegenstand, z. B. eine gefährliche Substanz, in dem Inspektionsobjekt 14 befindet, muss die verantwortliche Bedienperson mittels der Anzeigeeinheit 22 eine Sichtprüfung durchführen. Die Bedienperson entscheidet dann beispielsweise, ob das betreffende Inspektionsobjekt geöffnet werden und eine manuelle Inspektion durchgeführt werden muss.

Die Steuereinheit 18 weist Eingabemittel 24, wie beispielsweise eine Tastatur und/oder einzelne Eingabetasten, Steuerelemente, wie beispielsweise einen Joystick, eine Maus, einen Trackball oder ähnliches auf. Damit ist es der Bedienperson möglich, in üblicher und bekannter Weise Steuereingaben etc. vorzunehmen. In der zentralen Steuereinheit 18 befindet sich auch die für die hier beschriebenen Funktionen notwendige Hard- und Software eines üblichen und damit bekannten Datenverarbeitungssystems 20 (Computersystem). D. h. alle hier nachfolgend beschriebenen Funktionen können im Wesentlichen mittels eines an sich bekannten Datenverarbeitungssystems 20 in einer oder verteilt auf mehrere programmierbare Recheneinheiten umgesetzt werden. Das Datenverarbeitungssystem 20 ist dann im Wesentlichen zur Durchführung der beschriebenen Funktionen und Verfahren oder Teilen davon konfiguriert, üblicherweise programmiert.

Das Datenverarbeitungssystem 20 weist üblicherweise auf (nicht gezeigt): einen oder mehrere Prozessoren als zentrale Recheneinheit sowie internen Speicher und/oder externen Speicher, der für die Software nicht flüchtig und für Arbeitsdaten nach Art eines RAM (Random-Access-Memory) eingerichtet ist. Zur Interaktion mit funktionalen Bestandteilen der Vorrichtung ist das Datenverarbeitungssystem 20 über Kommunikationsschnittstellen beispielsweise zum Datennetzwerk 42 mit den einzelnen CT-Röntgeninspektionsanlagen 10.1, 10.2, 10.n verbunden. Als Ein-/Ausgabe-Schnittstelle zur Interaktion mit der Bedienperson dient der eine Bildschirm 22 oder mehrere Bildschirme als Anzeigeeinheit und die Tastatur 24 und/oder die Maus 25 als Eingabeeinheit.

Des Weiteren sei angemerkt, dass die hier beschriebenen Funktionen und Verfahren vollständige mittels eines Computerprogramms oder vollständig in Hardware sowie in jeder Mischform zwischen Hard- und Software zur Durchführung implementiert werden können. Für Bildverarbeitungsabläufe, wie beispielsweise bei der Bildprojektion eines gefährlichen Gegenstands (TIP) in ein Röntgenbild eines Inspektionsobjekts, können alle oder einzelne Verfahrensschritten auch durch auf Bildverarbeitung spezialisierte Hardware, wie graphische Bildverarbeitungsprozessoren (GPUs), in entsprechende Programmbefehle umgesetzt werden.

Zur Erzeugung von Test-Röntgenbildern zur Ausbildung oder Überprüfung einer Bedienperson ist eine Erzeugungseinrichtung 26 für CT-Test-Röntgenbilder vorgesehen, die eingerichtet ist, CT-Test-Röntgenbilder nach Art eines FTI, bevorzugt basierend auf kürzlich vor Ort kontrollierten Inspektionsobjekten zu erzeugen und zur Ausbildung und/oder Überprüfung einer Bedienperson anzuzeigen. Die mit dem hier im Zusammenhang mit der Figur 2 erläuterten TIP-Verfahren erzeugten CT-Test-Röntgenbilder können als pseudo-FTIs bezeichnet werden.

Figur 4 zeigt eine beispielhafte Ausführung der Erzeugungseinheit 26 mit einer Aktualisierungseinheit 32, die über eine Datenleitung 28 mit dem Datenverarbeitungssystem 20 der Steuereinheit 18 verbunden ist. Über die Datenleitung 28 werden der Aktualisierungseinheit 32 aktuelle im laufenden Betrieb der CT-Röntgeninspektionsanlagen 10.1, 10.2, 10.n des Kontrollsystems 1 übermittelt.

Die Aktualisierungseinheit 32 kann eingerichtet sein, erhaltene CT-Röntgenbilder von Inspektionsobjekten, die vor Ort im Kontrollsystem 1 inspiziert wurden, in einer Inspektionsobjektdatenbank 34 abzuspeichern. Die Aktualisierungseinheit 32 kann auch eingerichtet sein, die Inspektionsobjektdatenbank 34 so zu aktualisieren, dass ein dort gespeichertes Röntgenbild nicht älter als eine vorbestimmte zweite Zeitdauer ist und/oder ein dort gespeichertes Röntgenbild gelöscht oder für eine weitere Verwendung gesperrt wird, sobald es für eine vorbestimmte Anzahl, beispielsweise einmal, für die Erzeugung eines CT-Test-Röntgenbilds verwendet wurde. Dies hat den Vorteil, dass nur für den jeweiligen Zeitraum plausible Inhalte in den Inspektionsobjekten enthalten sind, die als Grundlage für ein FTI dienen; beispielsweise würde ein Koffer im Hochsommer, der für den Winter typische Gegenstände enthält, bereits aus diesem Grund auffallen.

In der Erzeugungseinheit 26 ist weiter eine Zielgegenstanddatenbank 36 vorgesehen, in der eine Bibliothek mit vierten CT-Daten T (und ggf. dritten CT-Daten TM) von Zielgegenständen gespeichert sind. Es sind Bilddaten solcher Zielgegenstände in der Zielgegenstanddatenbank 36 gespeichert, die durch das Kontrollsystem 1, aber besonders durch eine Bedienperson, in den Inspektionsobjekten aufgefunden werden sollen. Zielgegenstände können die hier bereits erläuterten Gegenstände sein.

Eine Bildprojektionseinheit 38 ist zur Projektion eines virtuellen Zielgegenstands aus der Zielgegenstandsdatendatenbank 36 in erste CT-Daten B der Inspektionsobjektdatenbank 34 zur Erzeugung von CT-Testbild-Daten FTI eingerichtet. Ein auf diese Weise erzeugtes Test-Röntgenbild kann von der Test-Röntgenbilderzeugungseinheit 26 über eine Datenleitung 30 dem Datenverarbeitungssystem 20 der Steuereinheit 18 zur Verfügung gestellt werden.

Das Datenverarbeitungssystem 20 ist eingerichtet, entsprechend der einschlägigen gesetzlichen Vorschriften beispielsweise ohne Hinweis oder Ankündigung im laufenden Betrieb einer Bedienperson zufällig ein 3D-Test-Röntgenbild oder 2D-Test-Röntgenbilder anzuzeigen, um den Ausbildungsstand, die Erkennungsfähigkeiten und gegebenenfalls die Aufmerksamkeit einer Bedienperson zu überprüfen.

Hinsichtlich der im Zusammenhang mit der Bildprojektion gefährlicher Gegenstände und/oder verbotener Substanzen geforderten Fähigkeiten des Systems sei zur Vermeidung von Wiederholungen auf die Verordnung (EU) Nr. 185/2010 vom 4. März 2010 zur Festlegung von detaillierten Maßnahmen für die Durchführung der gemeinsamen Grundstandards in der Luftsicherheit, insbesondere dort auf die Punkte 11.4. sowie 12.5. verwiesen. Der Vollständigkeit halber sei angemerkt, dass TIP auch eine Anforderung in entsprechenden Vorschriften außerhalb der Europäischen Gemeinschaft ist.

In der Erzeugungseinheit 26 kann weiter eine Test-Röntgenbilddatenbank 40 vorgesehen, in die von der Bildprojektionseinheit 38 erzeugte Test-Röntgenbilder abgespeichert werden können. Die Bildprojektionseinheit 38 kann dann weiter eingerichtet sein, Test-Röntgenbilder FTI in der Test-Röntgenbilderdatenbank 40 so zu verwalten, dass Test-Röntgenbilder, die älter als eine vorbestimmte Zeitdauer sind und/oder für eine bestimmte Anzahl, beispielsweise einmal, verwendet wurden, gelöscht oder für eine weitere Verwendung gesperrt werden. Damit kann eine große Anzahl von Test-Röntgenbildern in der Test-Röntgenbilderdatenbank 40 im laufenden Betrieb vorproduziert werden. Die Test-Röntgenbilderdatenbank 40 kann in ähnlicher Weise, wie im Zusammenhang mit der Inspektionsobjektdatenbank 34 beschrieben, aktualisiert werden. Im Ergebnis kann mit den Verwaltungsmaßnahmen erreicht werden, dass bei der Überprüfung von Bedienpersonen keine Test-Röntgenbilder verwendet werden, die den Bedienpersonen bereits bekannt sein könnten.

Das hier vorstehend beschriebene System kann Test-Röntgenbilder nach der Art von fiktiven Bedrohungsbildern (FTI) erzeugen, indem echte CT-Daten realer vor Ort inspizierter Inspektionsobjekten verwendet werden. Die Test-Röntgenbilder entsprechen im Wesentlichen Röntgenbildern realer Gepäckstücke, in die jeweils vorher ein reales Gefahrenobjekt eingebracht wurde; derartige Testbilder werden als sogenannte kombinierte Gefahrenbilder ("Combined Threat Image", CTI) bezeichnet. Auf diese Weise können Nachteile der Konzepte FTI und CTI vorteilhaft vermieden werden, besonders wird der laufende Betrieb des Kontrollsystems 1 nicht beeinträchtigt. Damit werden Bedienpersonen nicht an eine bestimmte Auswahl von Test-Röntgenbildern gewöhnt. Durch die laufende Aktualisierung der Test-Röntgenbilderdatenbank 40 ist es unwahrscheinlich, dass eine Bedienperson mit einem Test-Röntgenbild mehrmals konfrontiert wird. Falls nur solche Röntgenbilder für die Erzeugung von Test-Röntgenbildern verwendet werden, die von Inspektionsobjekten stammen, die vor Ort inspiziert wurden, wird sichergestellt, dass Gepäck- und/oder Versandstücke verwendet werden, die für den Kontrollort typisch sind bzw. für die aktuell am Kontrollort herrschenden Bedingungen typisch sind, wie beispielsweise die Jahreszeit. Im Wesentlichen wird sichergestellt, dass jede mögliche Auffälligkeit von Inspektionsobjekten, die typisch für den Kontrollort sind, auch Eingang in die Test-Röntgenbilder finden. Damit können die Test-Röntgenbilder einer Bedienperson nicht aufgrund von bewusst oder unbewusst wahrnehmbaren Abweichungen von ortstypischen Röntgenbildern "verdächtig" erscheinen.

Da mit dem neuen Erzeugungsverfahren für die Test-Röntgenbilder im Radonraum RR ausschließlich Artefakt-Sinogramm-Daten SA erzeugt werden, die nur den ersten CT-Daten B noch hinzuzufügende Metallartefakte A repräsentieren, werden die im Zusammenhang mit dem bekannten Verfahren diskutierten Auffälligkeiten der damit erzeugten Test-Röntgenbitder vermieden. Beim neuen Verfahren werden im Radonraum nur Sinogramm-Strahlen modifiziert, die Metallartefakte repräsentieren, die noch nicht in den ersten CT-Daten B enthalten sind; d.h. solche Metallartefakte, die auf das Metall des Zielobjekts und die auf die Wechselwirkung des Metalls des Zielobjekts mit bereits im Inspektionsobjekt enthaltenen Metallteilen zurückzuführen sind. Dies vermeidet, dass die ggf. bereits in den ersten CT-Daten B vorhandenen Metallartefakte zusätzlich verstärkt werden. Außerdem werden ausschließlich die neuen Artefakt-Sinogramm-Daten SA mittels der Radonrücktransformation zurück in den Bildraum transformiert. Die so im Bildraum vorliegenden CT-Artefakt-Daten A werden im Bildraum in die ersten CT-Daten B des Inspektionsobjekts eingefügt. D.h., beim neuen Verfahren werden nur neue Artefakte A berechnet und im Bildraum hinzugefügt, während im bekannten Verfahren Artefakte im Radonraum den ersten Sinogramm-Daten SB hinzugefügt werden und das gesamte modifizierte erste Röntgenbild durch Rücktransformation in den Bildbereich berechnet werden muss. D.h., das neue Verfahren muss keine modifizierten ersten Sinogramm-Daten SB* aus dem Radonraum in den Bildraum zurücktransformiert.

Beim neuen Verfahren werden schließlich im Bildraum in die ursprünglichen ersten CT-Daten B vierte CT-Daten der Zielgegenstand und die berechneten CT-Artefakt-Daten A eingefügt und die neuen fiktiven CT-Testbild-Daten FTI** erhalten, aus denen sich nach Bedarf 3D- und/oder 2D-Röntgenbilder des Inspektionsobjekts ableiten lassen.

Mit dem neuen Verfahren wird erreicht, dass neue fiktive Test-Röntgenbilder mit unverfälschten bereits vorhandenen Metallartefakte sowie den hinzugefügten Metallartefakten als auch dem eingefügten Zielgegenstand die gleiche Bildschärfe aufweisen wie ein ursprüngliches Röntgenbild basierend auf den ursprünglichen ersten CT-Daten B.

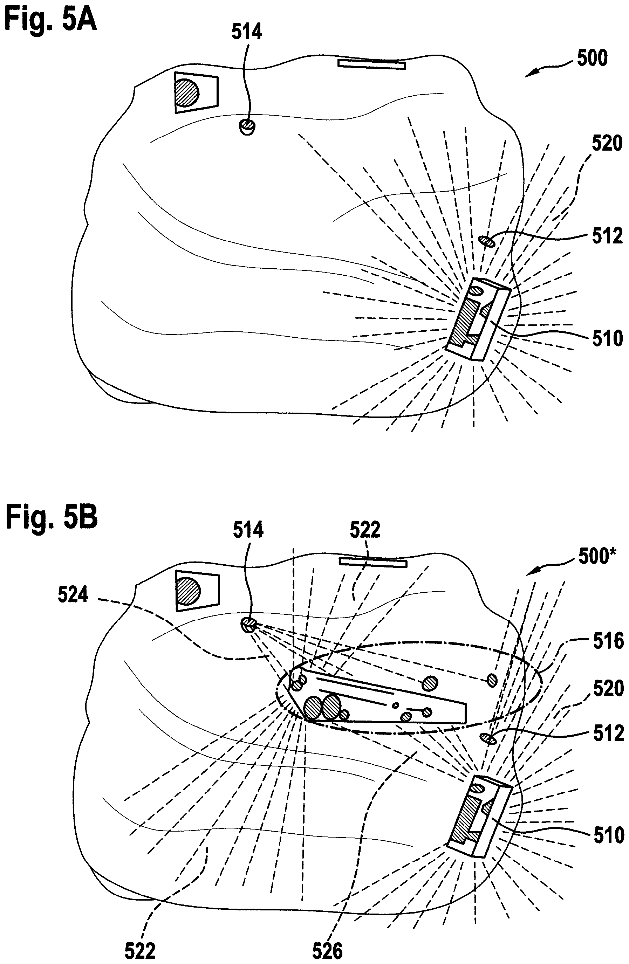

Figur 5A zeigt ein ursprüngliches 2D-Röntgenbild 500 eines Gepäckstücks als Beispiel für ein Inspektionsobjekt mit Metallartefakten 520, die von im Gepäckstück enthaltenen Metallteilen 510, 512, 514 verursacht sind. Die Metallartefakten 520 gehen im Wesentlichen auf das Metallteil 510 zurück und zeigen sich als vom Metallteil 510 ausgehende Strahlen.

Figur 5B zeigt das Röntgenbild der Figur 5A, in das ein Gefahrengestand als Zielgegenstand mit Metall(an)teilen 516 und die dazu erfindungsgemäß erzeugten Metallartefakte 522, 524, 526 eingefügt sind. Durch die fiktiv eingefügten Metallteile 516 sind Metallartefakte 522, die auf das Metallteil 516 alleine zurückgehen als auch Metallartefakte 524 und 526, die auf eine Wechselwirkung der bereits vorhandenen Metallteile 510, 512, 514 mit dem fiktiv eingefügten Metallteile 516 zurückzuführen sind, ebenfalls in das Röntgenbild einzufügen, damit dieses realistisch und plausibel ist. All dies leistet die vorliegende Erfindung.

## Patentansprüche

1. Erzeugungsverfahren für Test-Röntgenbildern, wobei das Verfahren aufweist:
Bereitstellen (S10) erster Computertomografie-, CT-, Daten (B), die ein Inspektionsobjekt repräsentieren, zweiter CT-Daten (BM), die im Inspektionsobjekt enthaltene Metallanteile repräsentieren, dritter CT-Daten (TM), die Metallanteile eines Zielgegenstands repräsentieren, der fiktiv in die ersten CT-Daten (B) eingefügt werden soll;
Transformieren (S20) der ersten, zweiten und dritten CT-Daten (B, BM, TM) aus dem Bildraum (BR) in entsprechende erste Sinogramm-Daten (SB), zweite Sinogramm-Daten (SBM) und dritte Sinogramm-Daten (STM) im Radonraum (RR);
Berechnen (S30, S40) von Artefakt-Sinogramm-Daten (SA) im Radonraum (RR), die Metall-Artefakte repräsentieren, die den ersten CT-Daten (B) aufgrund des einzufügenden Zielgegenstands hinzuzufügen sind, wobei für jeden Bildpunkt (p) in den zugehörigen Artefakt-Sinogramm-Daten (SA) ein Intensitätswert (SA(*p*)) entsprechend der folgenden Vorschrift berechnet wird:
| | | |
|---|---|---|
| (i) | *SA*(*p*) = (*q µ* - *q SB*(*p*))*,* | wenn STM(*p*) ≠ 0 und SBM(p) = 0, und |
| (ii) | SA(*p*) = 0, | anderenfalls, |
wobei *q* ein vorbestimmter Wert zwischen 0 und 1, und *µ* der Maximalwert in den zugehörigen zweiten Sinogramm-Daten (SBM) ist;
Rücktransformieren (S50) der Artefakt-Sinogramm-Daten (SA) aus dem Radonraum (RR) in den Bildraum (BR) in CT-Artefakt-Daten (A), welche die einzufügenden Artefakte repräsentieren; und
Einfügen der CT-Artefakt-Daten (A) in die ersten CT-Daten (B).

2. Erzeugungsverfahren gemäß Anspruch 1, wobei für *q* eines gilt von:
*q* hat einen Wert zwischen 0,02 und 0.06 ;
*q* hat einen Wert zwischen 0,03 und 0,04 ; und
*q* hat den Wert 0,04.

3. Erzeugungsverfahren gemäß Anspruch 1 oder 2, weiter mit Einfügen vierter CT-Daten (T), die den Zielgegenstand repräsentieren, in die ersten CT-Daten (B), um CT-Testbild-Daten (FTI) zu erhalten.

4. Erzeugungsverfahren gemäß Anspruch 2, weiter mit Ableiten von 3D-Röntgenbildern und/oder 2D-Röntgenbildern des Inspektionsobjekts aus den CT-Testbild-Daten (FTI).

5. Erzeugungsverfahren gemäß einem der Ansprüche 1-4, wobei das Einfügen von CT-Daten (A, T) in die ersten CT-Daten (B) aufweist:
gewichtetes Kombinieren eines Materialeigenschaftswerts, der einem bestimmten Voxel in den ersten CT-Daten (B) zugeordnet ist, in Abhängigkeit von einem jeweiligen Materialeigenschaftswert, der einem entsprechenden Voxel der einzufügenden CT-Daten zugeordnet ist, wobei dem entsprechenden Voxel und dem bestimmten Voxel sich entsprechende Raumkoordinaten zugeordnet sind.

6. Erzeugungsverfahren gemäß Anspruch 5, wobei das Einfügen von CT-Daten (A, T) in die ersten CT-Daten (B) weiter aufweist:
Klassifizieren mittels eines vorbestimmten Schwellwerts in den einzufügenden CT-Daten (T, A) von ersten Voxel, deren Materialeigenschaftswert unter dem vorbestimmten Schwellwert liegen, von zweiten Voxel, deren Materialeigenschaftswert in einem vorbestimmten Bereich über dem vorbestimmten Schwellwert liegen, und von dritten Voxel, deren Materialeigenschaftswert über diesem vorbestimmten Bereich liegen;
Ignorieren der CT-Daten der ersten Voxel;
gewichtetes Kombinieren des Materialeigenschaftswerts, der einem bestimmten Voxel der ersten CT-Daten (B) zugeordnet ist, mit dem Materialeigenschaftswert eines entsprechenden zweiten Voxel der einzufügenden CT-Daten; und
Ersetzen des Materialeigenschaftswerts, der einem bestimmten Voxel der ersten CT-Daten (B) zugeordnet ist, durch einen Materialeigenschaftswert der einem entsprechenden Voxel der einzufügenden CT-Daten zugeordnet ist,
wobei im Schritt des Verblendens und im Schritt des Ersetzens dem entsprechenden Voxel und dem bestimmten Voxel sich entsprechende Raumkoordinaten zugeordnet sind.

7. Erzeugungsverfahren gemäß einem der Ansprüche 1-6, wobei
die CT-Daten (B, BM, TM, T, A) wenigstens einen Wert für eine Materialeigenschaft aufweisen; oder
die CT-Daten (B, BM, TM, T, A) einen Wert für eine Materialeigenschaft in Form wenigstens eines von einen Absorptionskoeffizienten, einen Materialdichtewert, eine Kernladungszahl aufweisen.

8. Erzeugungsverfahren gemäß Anspruch 7, wobei die CT-Daten (B, BM, TM, T, A) ferner dreidimensionale, 3D-, Koordinaten zur Ortsbestimmung eines Volumenelements, dem der wenigstens eine Wert für eine Materialeigenschaft zugeordnet ist, aufweisen.

9. Computerprogrammprodukt mit einem Computerprogramm, das Softwaremittel zur Durchführung eines Verfahrens nach einem der Ansprüche 1 bis 8 aufweist, wenn das Computerprogramm in einem Automatisierungssystem ausgeführt wird.

10. Erzeugungseinrichtung (26; 26.1; 26.2) zur Erzeugung von Test-Röntgenbildern, wobei die Erzeugungseinrichtung (26; 26.1; 26.2) aufweist:
eine Inspektionsobjektdatenbank (34) zur Speicherung erzeugter erster CT-Daten (B) von Inspektionsobjekten (14); eine Zielgegenstanddatenbank (36) mit vierten CT-Daten (T) und ggf. dritten CT-Daten (TM) von Zielgegenständen; und eine Bildprojektionseinheit (38) zur Projektion der vierten CT-Daten (T) eines Zielgegenstands aus der Zielobjektdatenbank (36) in erste CT-Daten (B) aus der Inspektionsobjektdatenbank (34) zur Erzeugung von CT-Testbild-Daten (FTI), wobei die Bildprojektionseinheit (38) zur Durchführung eines Erzeugungsverfahrens gemäß einem der Ansprüche 1 bis 8 eingerichtet ist.

11. Erzeugungseinrichtung (26; 26.1; 26.2) gemäß Anspruch 10, wobei die Erzeugungseinrichtung zur Durchführung des Erzeugungsverfahrens ein Automatisierungssystem aufweist.

12. Zentrale Steuereinheit (18) für wenigstens eine Röntgeninspektionsanlage (10.1, 10.2, 10.n) zur zerstörungsfreien Inspektion eines Inspektionsobjekts (14), insbesondere eines Gepäckstücks oder sonstigen Versandstücks, mit einer Erzeugungseinrichtung (26; 26.1; 26.2) gemäß einem der Ansprüche 10 oder 11, wobei die Steuereinheit (18) bevorzugt über ein Kommunikationsnetzwerk (42) mit der wenigstens einen Röntgeninspektionsanlage (10.1, 10.2, 10.n) verbunden ist.

13. Zentrale Steuereinheit (18) gemäß Anspruch 12 mit wenigstens einer Anzeigeeinheit (22) zur Anzeige von Röntgenbildern eines aktuellen Inspektionsobjekts zur Sichtkontrolle durch eine Bedienperson oder zur Anzeige eines Test-Röntgenbilds.

14. Röntgeninspektionsanlage (10.1, 10.2, 10.n) zur zerstörungsfreien Inspektion eines Inspektionsobjekts (14), insbesondere eines Gepäckstücks oder sonstigen Versandstücks, die eine Zentrale Steuereinheit (18) gemäß Anspruch 12 oder 13 aufweist oder damit operativ und kommunikativ verbunden ist.

15. Gepäck- oder Versandstückkontrollsystem (1) mit wenigstens einer Röntgeninspektionsanlage (10.1, 10.2, 10.n) gemäß Anspruch 14.

## Claims

1. A production method for test X-ray images, the method comprising:
preparation (S10) of first computed tomography (CT) data (B) that represent an inspection object, second CT data (BM) that represent metal portions contained in the inspection object, and third CT data (TM) that represent metal portions of a target object, which is to be fictitiously inserted into the first CT data (B),
transformation (S20) of the first, second, and third CT data (B, BM, TM) from the image space (BR) into corresponding first sinogram data (SB), second sinogram data (SBM), and third sinogram data (STM) in the radon space (RR);
calculation (S30, S40) of the artifact sinogram data (SA) in the radon space (RR), which represent metal artifacts that are to be added to the first CT data (B) based on the target object that is to be inserted wherein for each pixel (p) in an associated artifact sinogram, an intensity value (SA(*p*)) is calculated in accordance with the following rules:
| | | |
|---|---|---|
| (i) | SA(*p*) = (*q µ* - *q* SB(*p*)) | if STM(*p*) ≠ 0 and SBM(p) = 0 and |
| (ii) | SA(*p*) = 0 | otherwise, |
where *q* is a predetermined value between 0 and 1 and *µ* is the maximum in the associated second sinogram data (SBM);
back-transformation (S50) of the artifact sinogram data (SA) from the radon space (RR) into the image space (BR) in CT artifact data (A), which represent the artifacts that are to be inserted; and
insertion of the CT artifact data (A) into the first CT data (B).

2. The production method according to claim 1, where for *q* applies one of:
*q* is a value between 0.02 and 0.06
*q* is a value between 0.03 and 0.04; and
*q* is the value of 0.04.

3. The production method according to claim 1 or 2, also comprising insertion of fourth CT data (T), which represent the target object, into the first CT data (B) in order to obtain CT test image data (FTI).

4. The production method according to claim 2, also comprising derivation of 3D X-ray images and/or 2D X-ray images of the inspection object from the CT test image data (FTI).

5. The production method according to one of claims 1-4, wherein the insertion of CT data (A, T) into the first CT data (B) comprises:
weighted combination of a material property value, which is associated with a particular voxel in the first CT data (B), as a function of a respective material property value, which is associated with a corresponding voxel of the CT data that are to be added, wherein corresponding spatial coordinates are associated with the corresponding voxel and the particular voxel.

6. The production method according to claim 5, wherein the insertion of CT data (A, T) into the first CT data (B) also comprises:
classification, by means of a predetermined threshold in the CT data (T, A) to be added, of first voxels whose material property values lie below the predetermined threshold, second voxels whose material property values lie in a predetermined range above the predetermined threshold, and third voxels, whose material property values lie above this predetermined range;
ignoring of the CT data of the first voxels;
weighted combination of the material property value, which is associated with a particular voxel of the first CT data (B), with the material property value of a corresponding second voxel of the CT data that are to be inserted; and
replacement of the material property value, which is associated with a particular voxel of the first CT data (B), with a material property value, which is associated with a corresponding voxel of the CT data that are to be added,
wherein in the step of the blending and in the step of the replacement, corresponding spatial coordinates are associated with the corresponding voxel and the particular voxel.

7. The production method according to one of claims 1-6, wherein
the CT data (B, BM, TM, T, A) comprise at least one value for a material property; or
the CT data (B, BM, TM, T, A) comprise a value for a material property in the form of at least one of an absorption coefficient, a material density value, and an atomic number.

8. The production method according to claim 7, wherein the CT data (B, BM, TM, T, A) further comprise three-dimensional, 3D-, coordinates for determining the position of a voxel, which is associated with the at least one value for a material property.

9. A computer program product having a computer program, which has software means for carrying out a method according to one of claims 1 to 8 when the computer program is run in an automation system.

10. A production apparatus (26; 26.1; 26.2) for producing test X-ray images, wherein the production apparatus (26; 26.1; 26.2) comprises:
an inspection object database (34) for storing produced first CT data (B) of inspection objects (14); a target object database (36) with fourth CT data (T) and possibly third CT data (TM) of target objects; and an image projection unit (38) for projecting the fourth CT data (T) of a target object from the target object database (36) into first CT data (B) from the inspection object database (34) in order to produce CT test image data (FTI), wherein the image projection unit (38) is configured to carry out a production method according to one of claims 1 to 8.

11. The production apparatus (26; 26.1; 26.2) according to claim 10, wherein the production apparatus has an automation system for carrying out the production method.

12. A central control unit (18) for at least one X-ray inspection unit (10.1, 10.2, 10.n) for nondestructive inspection of an inspection object (14), particularly of a luggage item or other package, having a production apparatus (26; 26.1; 26.2) according to one of claims 10 or 11, wherein the control unit (18) is preferably connected to the at least one X-ray inspection unit (10.1, 10.2, 10.n) via a communication network (42).

13. The central control unit (18) according to claim 12, having at least one display unit (22) for displaying X-ray images of a current inspection object for visual inspection by an operator or for displaying a test X-ray image.

14. An X-ray inspection unit (10.1, 10.2, 10.n) for nondestructive inspection of an inspection object (14), particularly of a luggage item or other package, which has a central control unit (18) according to claim 12 or 13 or is operatively and communicatively connected thereto.

15. A luggage or package screening system (1) having at least one X-ray inspection unit (10.1, 10.2, 10.n) according to claim 14.

## Revendications

1. Procédé de génération pour images test radiographiques, le procédé comportant :
mise à disposition (S10) de premières données (B) de tomographie par ordinateur, CT, représentant un objet d'inspection, de deuxièmes données CT (BM) représentant des parts de métal contenues dans l'objet d'inspection, de troisièmes données CT (TM) représentant les parts de métal d'un objet cible qui doit être fictivement inséré dans les premières données CT (B) ;
transformation (S20) des premières, deuxièmes et troisièmes données CT (B, BM, TM) de l'espace image (BR) en des premières données de sinogramme (SB), des deuxièmes données de sinogramme (SBM) et des troisièmes données de sinogramme (STM) correspondantes dans l'espace radon (RR) ;
calcul (S30, S40) de données de sinogramme artefact (SA) dans l'espace radon (RR) qui représentent des artefacts métalliques devant être ajoutés aux premières données CT (B) en raison de l'objet cible à insérer, cependant que, pour chaque pixel (*p*) dans les données de sinogramme artefact (SA) associées, une valeur d'intensité (SA(*p*)) est calculée conformément à la spécification suivante :
| | | |
|---|---|---|
| (i) | SA(*p*) + (*q µ* - q SB(*p*)) | quand STM(*p*) ≠ 0 et SBM(*p*) = 0, et |
| (ii) | SA(*p*) = 0 | quand ce n'est pas le cas, |
cependant que *q* est une valeur prédéterminée entre 0 et 1, et *µ* est la valeur maximale dans les deuxièmes données de sinogramme (SBM) associées ;
retransformation (S50) des données de sinogramme artefact (SA), depuis l'espace radon (RR) dans l'espace image (BR), en des données artefact CT (A) qui représentent les artefacts à insérer ; et
insertion des données artefact CT (A) dans les premières données CT (B).

2. Procédé de génération selon la revendication 1, cependant que, pour *q*, il est entendu que soit :
*q* a une valeur entre 0,02 et 0,06 ;
*q* a une valeur entre 0,03 et 0,04 ; et
*q* a la valeur 0,04.

3. Procédé de génération selon une des revendications de 1 ou 2, comprenant en outre l'insertion de quatrièmes données CT (T), représentant l'objet cible, dans les premières données CT (B) afin d'obtenir des données d'image test CT (FTI).

4. Procédé de génération selon la revendication 2, comprenant en outre la déduction d'images radiographiques 3D et/ou d'images radiographiques 2D de l'objet d'inspection à partir des données d'image test CT (FTI).

5. Procédé de génération selon une des revendications de 1 à 4, cependant que l'insertion de données CT (A, T) dans les premières données CT (B) comporte :
combinaison pondérée d'une valeur de propriété de matériau affectée à un voxel déterminé dans les premières données CT (B), en fonction d'une valeur de propriété de matériau respective affectée à un à un voxel correspondant des données CT à insérer, cependant que, au voxel correspondant et au voxel déterminé, des coordonnées spatiales se correspondant entre elles sont affectées.

6. Procédé de génération selon la revendication 5, cependant que l'insertion de données CT (A, T) dans les premières données CT (B) comporte en outre :
classification, au moyen d'une valeur seuil prédéterminée, dans les données CT à insérer (T, A), de premiers voxels dont la valeur de propriété de matériau est située en-dessous de la valeur seuil prédéterminée, de deuxièmes voxels dont la valeur de propriété de matériau est située dans une plage prédéterminée au-dessus de la valeur seuil prédéterminée, et de troisièmes voxels dont la valeur de propriété de matériau est située au-dessus de cette plage prédéterminée :
non-prise en considération des données CT des premiers voxels ;
combinaison pondérée de la valeur de propriété de matériau affectée à un voxel déterminé des premières données CT (B), avec la valeur de propriété de matériau respective d'un deuxième voxel correspondant des données CT à insérer, et
remplacement de la valeur de propriété de matériau affectée à un voxel déterminé des premières données CT (B) par une valeur de propriété de matériau affectée à un voxel correspondant des données CT à insérer,
cependant que, à l'étape du masquage et à l'étape du remplacement, des coordonnées spatiales se correspondant entre elles sont affectées au voxel correspondant et au voxel déterminé.

7. Procédé de génération selon une des revendications de 1 à 6, cependant que,
les données CT (B, BM, TM, T, A) comportent au moins une valeur pour une propriété de matériau ; ou
les données CT (B, BM, TM, T, A) comportent une valeur pour une propriété de matériau sous forme d'au moins un d'un coefficient d'absorption, d'une valeur de densité de matériau, d'un numéro atomique.

8. Procédé de génération selon la revendication 7, cependant que les données CT (B, BM, TM, T, A) comportent en outre des coordonnées tridimensionnelles, 3D, pour la détermination de l'emplacement d'un élément de volume auquel la au moins une valeur est affectée pour une propriété de matériau.

9. Produit programme d'ordinateur ayant un programme d'ordinateur qui comporte des moyens logiciels pour l'exécution d'un procédé selon une des revendications de 1 à 8 quand le programme d'ordinateur est exécuté dans un système d'automatisation.

10. Dispositif de génération (26; 26.1; 26.2) destiné à la génération d'images test radiographiques, cependant que le dispositif (26; 26.1; 26.2) comporte :
une banque de données d'objets d'inspection (34) pour la mémorisation de premières données CT (B) générées d'objets d'inspection (14) ; une banque de données d'objets cible (36) renfermant des quatrièmes données CT (T) et éventuellement des troisièmes données CT (TM) d'objets cible ; et une unité de projection d'image (38) pour la projection des quatrièmes données CT (T) d'un objet cible depuis la banque de données d'objets cible (36) en des premières données CT (B) depuis la banque de données d'objets d'inspection (34) pour la génération de données d'image test CT (FTI), cependant que l'unité de projection d'image (38) est conçue pour la réalisation d'un procédé de génération selon une des revendications de 1 à 8.

11. Dispositif de génération (26; 26.1; 26.2) selon la revendication 10, cependant que le dispositif de génération comporte pour la réalisation du procédé de génération un système d'automatisation.

12. Unité centrale de commande (18) destinée à au moins une installation d'inspection radiographique (10.1, 10.2, 10.n) pour l'inspection non destructive d'un objet d'inspection (14), en particulier d'une pièce de bagage ou autre pièce transportée, dotée d'un dispositif de génération (26; 26.1; 26.2) selon une des revendications 10 ou 11,
cependant que l'unité de commande (18) est, de préférence par l'intermédiaire d'un réseau de communication (42), reliée avec la au moins une installation d'inspection radiographique (10.1, 10.2, 10.n).

13. Unité centrale de commande (18) selon la revendication 12, dotée d'au moins une unité d'affichage (22) pour l'affichage d'images radiographiques d'un objet d'inspection actuel pour le contrôle visuel par un opérateur ou pour l'affichage d'une image test radiographique.

14. Installation d'inspection radiographique (10.1, 10.2, 10.n) pour l'inspection non destructive d'un objet d'inspection (14), en particulier d'une pièce de bagage ou autre pièce transportée, qui est dotée d'une unité centrale de commande (18) selon la revendication 12 ou 13 ou qui est reliée de manière opérationnelle et communicative avec elle.

15. Système de contrôle de pièce de bagage ou de pièce transportée (1) muni d'au moins une installation d'inspection radiographique (10.1, 10.2, 10.n) selon la revendication 14.
